# EUROPEAN PATENT SPECIFICATION

(11) **EP 1 997 483 B1**
(45) Date of publication and mention of the grant of the patent: **18.07.2018**
(21) Application number: 06761631.8
(22) Date of filing: 27.07.2006
(51) Int. Cl.: A61K 9/72, A61K 38/18, A61P 9/10, A61K 9/08

(54) **RH-EPO NASAL FORMULATIONS WITH LOW SIALIC ACID CONCENTRATION FOR THE TREATMENT OF DISEASES OF THE CENTRAL NERVOUS SYSTEM**
NASALE RH-EPO-FORMULIERUNGEN MIT NIEDRIGER SIALINSÄUREKONZENTRATION ZUR BEHANDLUNG VON ERKRANKUNGEN DES ZENTRALEN NERVENSYSTEMS
FORMULATIONS NASALES D'EPORH A FAIBLE TENEUR EN ACIDE SIALIQUE UTILISEES DANS LE TRAITEMENT DE MALADIES DU SYSTEME NERVEUX CENTRAL

(30) Priority: 22.07.2005 CU 1382005
(43) Date of publication of application: 03.12.2008
(73) Proprietor: Centro de Investigación y Desarrollo de Medicamentos (CIDEM), C.P. 10400 Ciudad de la Habana (CU)
(72) Inventor: MUÑOZ CERNADA, Adriana, C.P.:10700 Ciudad de La Habana (CU); GARCÍA RODRÍGUEZ, Julio César, C.P.:17200 Ciudad de La Habana (CU); NUÑEZ FIGUEREDO, Yanier, Ciudad de La Habana C.P.:14000 Ciudad de La Habana (CU); PARDO RUIZ, Zenia, C.P.: 11500 Ciudad de La Habana (CU); GARCÍA SELMAN, Jorge Daniel, Ciudad de La Habana C.P.: 10600 (CU); SOSA TESTÉ , Iliana, Ciudad de La Habana C.P.: 10600 (CU); CURBELO RODRÍGUEZ, David, Ciudad de La Habana C.P.: 11600 (CU); CRUZ RODRÍGUEZ, Janette, Ciudad de La Habana C.P.: 13600 (CU); SUBIROS MARTÍNEZ, Nelvys, Ciudad Habana C.P.: 32700 (CU)
(74) Representative: V.O.
(86) International application number: PCT/CU2006/000007
(87) International publication number: WO 2007/009404

(56) References cited:
- EP-A1- 1 093 818
- WO-A1-91/11200
- WO-A2-2004/002402
- WO-A2-2005/004895
- GB-A- 2 177 914
- US-A1- 2004 122 216
- SHIMODA N ET AL: "EFFECTS OF DOSE, PH AND OSMOLARITY ON INTRANASAL ABSORPTION OF RECOMBINANT HUMAN ERYTHROPOIETIN IN RATS", BIOLOGICAL & PHARMACEUTICAL BULLETIN (OF JAPAN), PHARMACEUTICAL SOCIETY OF JAPAN, TOKYO, JP, vol. 18, no. 5, 1 May 1995 (1995-05-01), pages 734-739, XP000511935, ISSN: 0918-6158
- WANG X ET AL: "The nonerythropoietic asialoerythropoietin protects against neonatal hypoxia-ischemia as potently as erythropoietin", JOURNAL OF NEUROCHEMISTRY, WILEY INTERSCIENCE, NEW YORK, NY, US, vol. 91, no. 4, 1 January 2004 (2004-01-01), pages 900-910, XP003004626, ISSN: 0022-3042, DOI: 10.1111/J.1471-4159.2004.02769.X
- YU Y P ET AL: "Intranasal recombinant human erythropoietin protects rats against focal cerebral ischemia", NEUROSCIENCE LETTERS, LIMERICK, IE, vol. 387, no. 1, 14 October 2005 (2005-10-14), pages 5-10, XP027654048, ISSN: 0304-3940 [retrieved on 2005-10-14]
- ERBAYRAKTAR S. ET AL.: 'Asialoerythropoietin is a nonerythropoietic cytokine with broad neuroprotective activity in vivo' PNAS vol. 100, no. 11, 27 May 2003, pages 6741 - 6747, XP009070746
- SOSA-TESTE ET AL.: 'Intranasal administration of recombinant human erythropoietin exerts neuroprotective effects on post-ischemic brain injury in mongolian gerbils' PHARMACOLOGYONLINE, [Online] vol. 1, pages 100 - 112, XP003005912 Retrieved from the Internet: <URL:http://www.pharmacologyonlines.unisa.i t/archivies/2006/volue1/8_sosaTeaste.pdf>

## Description

### OBJECT OF THE INVENTION

This invention is related to the biopharmaceutical industry and in particular to the production of an intranasal medication of rhEPO with low content of (basic) sialic acid starting from Chinese hamster's ovary cells by way of genetic engineering for the treatment of cerebrovascular, neurodegenerative and psychiatric diseases.

### BACKGROUND OF THE INVENTION

Erythropoietin (EPO) is a glycoprotein hormone whose molecular weight is within the range of 34 kDa; the protein fraction consists of approximately 166 amino acids; it is produced in the cells of the kidneys, the liver and of the central nervous system and it is involved in the proliferation, differentiation and maturation of the erythrocytes and of other hematopoietic cells.

In 1998, Sakanaka and collaborators reported the neuron-protecting properties of rhEPO against ischemic injury *in vivo* after the occlusion of the common carotid artery, a model of global ischemia followed by infusion of rhEPO into the lateral ventricles of gerbils. Those authors observed a reduction of ischemic injury on the CA1 hippocampal neurons.

The neuron-protecting effects of rhEPO can be due to different factors, including: antagonism of glutamate-induced cytotoxicity, increase in the expression of antioxidant enzymes, decrease in the formation of nitric oxide (NO) mediated by free radicals, normalization of brain blood flow, influence on the liberation of neurotransmitters, promotion of neoangiogenesis, inhibition of the apoptosis induced by excitotoxin or nitric oxide, increase in the expression of antiapoptotic genes, neurotropic effect, decrease in neuron excitability, brain anti-inflammatory effect, inhibition of the apoptosis induced by kainato and the production of pro-inflammatory cytokines, angiogenic and neurogenic effects. Furthermore, rhEPO contributes to ischemic preconditioning. As a whole, these considerations can justify the use of rhEPO in the treatment of cerebrovascular, neurodegenerative, psychiatric diseases.

The neuron-protecting properties of rhEPO can prevent or revert neuron death, an occurrence characterizing many diseases of the central nervous system, such as ischemia, neural trauma and neurodegenerative and neuron-inflammatory diseases.

It has also been described that the neurodegenerative processes contribute to trigger the physio-pathology of schizophrenia. This is why the neuron-protecting drugs can be an effective therapeutic alternative for the treatment of this disease.

Once that the organism has been exposed to certain pathological conditions, such as hypoglycemia or strong neuron depolarization, or due to the generation of oxygen-reactive species by the mitochondria, the expression of rhEPO increases notably.

In other respect, inflammation processes increase brain injury through different mechanisms, and they even directly inhibit the production of EPO locally. In fact, even though rhEPO is produced endogenously in the brain after ischemia, its expression is notably inhibited by inflammatory cytokines. This inhibition reduces the neuron-protecting capacity of rhEPO and justifies the exogenous administration of rhEPO as especially beneficial for the treatment of cerebrovascular, neurodegenerative or psychiatric diseases.

Additionally, it is well-known that the affinity of the rhEPO molecule for its receiver (and, hence, the effectiveness of the physiological response to this molecule) is inversely related to the percentage of sialic acid associated to it.

The neuron-protective effect of rhEPO administered by the intravenous way to murine models of brain ischemia [LM Brines and cols, PNAS97 (19):10526-10531, 2000] reduces the injury caused by ischemia between 50 and 75%, and a similar effect occurs in humans (WO 0354475). However, the clinical and experimental results of using acid recombinant human erythropoietin (rhEPO) have not as yet valued the potentially negative medium-term impact of using this molecule on the induction of erythropoiesis, since, given the low permeability of the blood brain barrier (BBB) and the low affinity of the rhEPO receivers in the CNS, relatively large quantities of rhEPO are required for a relatively long time to achieve an effective pharmacological effect. Under these conditions, one runs the risk of increased viscosity of the blood and of increased possibility of thrombolytic events leading to cerebral ischemia. These negative consequences of the application of acid rhEPO have been reported as complications in nephritic patients treated with this molecule.

Studies have been conducted in order to develop methods that increase the permeability of the BBB, which is considered to be a critical step in the incorporation of substances to brain tissues (US 5260308; WO 8901343; US 4801575; US 5442043; JP 57146710 and JP 01149718). Many of these methods involve the so-called carriers (US 5442043) forming a peptidic conjugate or they use chimeric peptides (US 48015575) for the incorporation of the substances to the brain. The preparation of liposomes containing erythropoietin has been reported (JP 08231417).

The use of the intranasal way to cause molecules to reach the CNS has been reported for gangliosides (US 4639437), as well as for neuronal therapeutic agents reaching the brain through their absorption by the mucous membrane of the nose and their subsequent transportation through the neural olfactory way (EP 504263).

There exist several patents related to the protection of liquid formulations of rhEPO, the most representative of which are listed next:
Liquid preparations in which the active principle used is rhEPO, and in which the protein is in all cases presented in injectable form (e.g.: CIPO-2041989; CIPO-2353553). This administration way indicates that the rhEPO used has been of the acid type, that is, more than 40 % of the molecule would be covered with sialic acid; otherwise it would have been inactivated by the hepatic enzymes and it would not be clinically effective.

The patent CIPO-2074820 uses the intranasal way for the administration of the protein. In this case, cyclodextrins are used as a component of the formulation and, likewise, a systemic absorption of the rhEPO is pursued, so that the protein has also to be acid.

There exist scientific publications referring to the use of bioadhesive agents to increase the residence time of proteins in the nasal cavity Polireddy D and cols, International Journal of Pharmaceutics (127):115 - 133, 1996]; however, there exist no reports on the use of bioadhesive agents in formulations containing rhEPO.

The patent CIPO-2353553 justifies the exogenous administration of rhEPO for the treatment of cerebral ischemia, but, just the same as in previous cases, the pursuit is that the protein passes directly or indirectly to the blood stream in order to be effective, so that we are equally in the presence of rhEPO with high content of sialic acid.

The patent CIPO-2408685 covers different formulations of rhEPO, but in this case they are intended for the treatment of anemia, so that it is acid rhEPO, since basic rhEPO cannot promote proliferation, differentiation and maturation of erythrocytes.

The patent CIPO-2437333 specifies that the protected formulations start from alpha erythropoietin, i.e., from acid rhEPO.

In all the cases exposed previously and revised in the literature reference is made to acid rhEPO, whereas the patent we are applying for is intended for the protection of liquid formulations starting from basic rhEPO.

Wang et al., Journal of Neurochemistry, 91:4 p.900-910 (2004) relates to neuroprotective effects associated with intraperitoneal administration of asialoerythropoietin.

Erbayraktar et al., PNAS, 100:11, p. 6741-6747 (2003) relates to neuroprotective effects associated with intravenous administration of asialoerythropoietin.

### DESCRIPTION OF THE INVENTION

During the production of rhEPO, the glycoprotein is obtained with different contents of sialic acid. Protein having less than 40% of its molecule protected with (basic) sialic acid is normally eliminated, since it is not biologically active by the systemic way. This basic rhEPO, with a low content of sialic acid, represents 70% of the total production of rhEPO. Surprisingly, we have found that intranasal administration of basic rhEPO can have higher therapeutic values than the acid rhEPO; the results of this invention allow to take advantage of the basic isoforms of rhEPO, which at the moment are not recovered and are of easy acquisition. This represents a great economic advantage because it allows to recover what that at the moment constitutes a waste of the production of rhEPO.

The invention provides a formulation according to claim 1.

The invention also provides a formulation for use according to claim 7.

This formulation can be used for the treatment and/or prevention of diseases of the central nervous system including cerebrovascular, neurodegenerative and psychiatric disorders, on an ambulatory basis at the level of the primary health care system. The advantages of the proposed solution include the employment of rhEPO with low content of sialic acid for therapeutic ends allowing greater protective effects with smaller doses. A quick arrival of the therapeutic agent to the action place is achieved, and this is critical to attain satisfactory therapeutic effects in the presence of cerebral hypoxia. In the specific case of cerebrovascular diseases, in the acute phase it is necessary to begin with the therapeutic intervention before certain processes of the cascade ischemia have happened. This is called the period of therapeutic window.

Intranasal administration of basic rhEPO eliminates the risk of inducing erythropoiesis. An increment in the erythrocyte concentration would increase blood viscosity, thereby endangering reperfusion after an episode of occlusion or hemorrhage, so that the cerebral blood flow would diminish and therefore the arrival of oxygen and nutrients to brain tissues would be hindered.

The intranasal way has greater advantages than other administration forms, allowing quick access to the brain; it is less invasive than the endovenous or the intracerebroventricular ways, and a percent of the rhEPO can reach the cerebrospinal fluid without having to pass before to the blood stream; this avoids first hepatic step metabolism, thereby preventing its consequent inactivation. Intranasal application is not traumatic and eliminates surgical risks and other possible implications given by the traumatic ways. It constitutes an alternative way of access to the brain without damaging the BBB. The use of an administration way alternative to the vascular way assures the arrival of the molecule to the areas of the central nervous system with poor or null blood irrigation, by diffusion through the interstitial fluid.

Keeping in mind that one of the main limitations of intranasal administration is the quick cilium-induced mucus clearing, this invention resorts to the strategy of using bioadhesive polymers that increase the residence time of rhEPO in the nasal cavity. During the development of the formulation, other auxiliary substances or recipients were also incorporated, such as preservatives, tensioactive agents, pH regulators, isotonic agents and protein stabilizers in order to obtain a formulation with physical, chemical and microbiological stability that maintains its therapeutic properties throughout time.

The rhEPO used contained in its molecule less than 40% of sialic acid (that is, basic rhEPO) with concentration ranging from 0,5 to 2 mg/mL. The formulations hereby presented are liquid, colorless, transparent preparations free from mechanical impurities, with apparent viscosity ranging from 10 to 250 mPas. As bioadhesive polymers, hydroxypropylmethylcellulose (HPMC) in concentrations from 0,4 to 0,9 %, hydroxypropylcellulose (HPC) from 0,2 to 0,8 % and methyl cellulose (MC) from 0,25 to 0,5 % were employed.

The pH was adjusted to a range from 6,0 to 7,5 using mainly phosphate and citrate buffers in concentrations from 20 to 100 mM/L.

The osmotic pressure can be regulated with different isotonic agents, for example: sodium chloride, mannitol, sorbitol, glucose, among others, in a range from 0,05 to 10 g/L.

The preservative agents used were: benzalkonium chloride in concentrations from 0,01 to 0,02 % in combination with disodium EDTA in concentrations within the range of 0,01%, chlorobutanol from 0,3 to 0,5%, methylparaben from 0,02 to 0,035 % and propylparaben from 0,01 to 0,02 %.

In order to avoid the adherence of the protein to the walls of the container, non-ionic non-ionic surfactants were used, such as polyethylenesorbitan laureate (Tween 20 to 80), cremophor RH 40, sorbitan trioleate (spam 35 to 80), in a range of concentrations from 0,01 to 1 g/L.

As protein stabilizers, different aminoacids were used, among others: L-tryptophane, L-leucine, L-arginine hydrochloride and L-histidine hydrochloride in a range of concentrations between 0,1 and 10 mg/mL. The solvent used in all the cases was injection water.

Tests of irritability of the nasal mucous membrane were conducted in rats, and no indications of irritation were found at this level. This, together with the excellent tolerance of the product, provides toxicological validation for the employment of basic rhEPO in humans.

Stability studies showed an appropriate behavior of protein concentrations in time. Upon application of the Lowry method (± 10%), the pH stayed within the limits established.

The invention also provides a recombinant human erythropoietin for use according to claim 9.

### WORKING EXAMPLES

### Example 1:

### Purification of rhEPO with low content of sialic acid:

For obtaining basic erythropoietin with sialic acid content less than 40%, three basic chromatographic steps were carried out, starting from a material of great complexity and with a great quantity of contaminating proteins, since this material contains 5% of bovine fetal serum. An additional step is then performed to obtain the isoforms of interest.

For the diafiltration and concentration of basic rhEPO an ultrafiltration system at laboratory scale (SARTOFLOW SLICE 200) was used, coupled to a peristaltic bomb (IP55 Watson-Marlow). A Hydrosart membrane of 10 kDa was used. The work was performed at a flow of 1,9 ± 0,25 mL/min, a feeding pressure (P1) of 2,9 ± 0,25 bar, a retained fraction pressure (P2) of 0,65 ± 0,1 bar and a transmembrane (TM) pressure of 1,8 ± 0,2 bar. For the determination of protein concentration, an Ultrospec TM 3100 spectrophotometer was used at λ = 280 nm. For the diafiltration process, an intranasal buffer solution of NaH₂PO₄ and Na₂HPO₄ at a pH of 6,0 and a conductivity of 3,25 ms/cm was used. The pH was controlled by using a Sartorius pH meter and an Omega conductimeter. The yield of the process is found in the range of 75 - 95 %.

**Table 1, shows the results of the process:**

| Time (h) | pH | Conductivity (ms/cm) | Concentratio n (mg/mL) | Volume of intranasal solution incorporated (mL) | Real volume (mL) |
|---|---|---|---|---|---|
| 0 | 7,12 | 14,29 | 1,72 | - | 290,0 |
| 6,0 | 6,5 | 4,5 | 1,3 | 800 | - |
| 12 | 6,0 | 3,5 | 2,1 | 1100,0 | 210,0 |

### Example 2:

**Nasal formulation of rhEPO with low content of sialic acid that uses HPMC as bioadhesive polymer, histidine hydrochloride as stabilizer, Tween 80 as non-ionic surfactants and benzalkonium chloride as preservative.**

### Preparation method:

The preparation of the solution starts from a volume of injection water representing 30% of the total volume of the formulation. This volume is used to dissolve the preservative, the buffer and the isotonic agent. Apart from this, in a container of appropriate capacity, a volume of injection water equal to 15 % of the total volume of the formulation is placed and warmed to a temperature ranging from 85 to 95°C, and the polymer is dispersed with strong, constant stirring. Once the polymer is moistened, the previously prepared solution is incorporated to the same while shaking vigorously until total homogenization. Shaking is then reduced in order to incorporate the corresponding volume of basic rhEPO, the non-ionic surfactant and the protein stabilizer. Finally, the volume is completed with injection water until the 100% of the formulation's final volume. The formulation is subsequently filtered through a membrane of cellulose acetate of 0,2 µm and the pH is tested to verify that it keeps within the established range.

The composition of the finished preparation is as follows:

| **Component** | **Proportion** |
|---|---|
| Basic rhEPO | 0,8 mg/mL |
| Hydroxypropylmethylcellulose | 5,0 mg/mL |
| Histidine hydrochloride | 1,0 mg/mL |
| Dihydrogen sodium phosphate | 2 mg/mL |
| Hydrogen disodium phosphate | 0,7 mg/mL |
| Tween 80 | 0,25 mg/mL |
| Sodium chloride | 7,4 mg/mL |
| Disodium ethylenediamine | 0,1 mg/mL |
| tetraacetic acid | |
| Benzalkonium chloride | 0,2 mg/mL |
| Injection water | q.s. 1 ml |

### Example 3:

**Nasal formulation of rhEPO with low content of sialic acid similar to the former example, but using methylcellulose as bioadhesive polymer.**

### Preparation method: similar to example 2.

The composition of the finished preparation is as follows:

| **Component** | **Proportion** |
|---|---|
| Basic rhEPO | 0,8 mg/mL |
| Methylcellulose | 3,0 mg/mL |
| Histidine hydrochloride | 1,0 mg/mL |
| Dihydrogen sodium phosphate | 2 mg/mL |
| Hydrogen disodium phosphate | 0,7 mg/mL |
| Tween 80 | 0,25 mg/mL |
| Sodium chloride | 7,4 mg/mL |
| Disodium ethylenediamine | 0,1 mg/mL |
| tetraacetic acid | |
| Benzalkonium chloride | 0,2 mg/mL |
| Injection water | q.s. 1 ml |

### Example 4:

**Nasal formulation of rhEPO with low content of sialic acid similar to example 2, but using Tween 20 as the non-ionic surfactant.**

### Preparation method: similar to example 2.

The composition of the finished preparation is as follows:

| **Component** | **Proportion** |
|---|---|
| Basic rhEPO | 0,8 mg/mL |
| Hydroxypropylmethylcellulose | 5,0 mg/mL |
| Histidine hydrochloride | 1,0 mg/mL |
| Dihydrogen sodium phosphate | 2 mg/mL |
| Hydrogen disodium phosphate | 0,7 mg/mL |
| Tween 20 | 0,20 mg/mL |
| Sodium chloride | 7,4 mg/mL |
| Disodium ethylenediamine tetraacetic acid | 0,1 mg/mL |
| Benzalkonium chloride | 0,2 mg/mL |
| Injection water | q.s. 1 ml |

### Example 5:

**Nasal formulation of rhEPO with low content of sialic acid similar to the former example, but with 1,2 mg/ml of the active principle and without histidine hydrochloride as protein stabilizer.**

### Preparation method: similar to example 2, but without using histidine hydrochloride.

The composition of the finished preparation is as follows:

| **Component** | **Proportion** |
|---|---|
| Basic rhEPO | 1,2 mg/mL |
| Hydroxypropylmethylcellulose | 5,0 mg/mL |
| Dihydrogen sodium phosphate | 2 mg/mL |
| Hydrogen disodium phosphate | 0,7 mg/mL |
| Tween 20 | 0,20 mg/mL |
| Sodium chloride | 7,7 mg/mL |
| Disodium ethylenediamine tetraacetic acid | 0,1 mg/mL |
| Benzalkonium chloride | 0,2 mg/mL |
| Injection water | csp 1 ml |

### Example 6:

**Nasal formulation of rhEPO with low content of sialic acid similar to example 2, but with 1.2 mg/ml of active principle and without histidine hydrochloride as protein stabilizer.**

### Preparation method: similar to example 2, but without using histidine hydrochloride.

The composition of the finished preparation is as follows:

| **Component** | **Proportion** |
|---|---|
| Basic rhEPO | 1.2 mg/mL |
| Hydroxypropylmethylcellulose | 5.0 mg/mL |
| Dihydrogen sodium phosphate | 2 mg/mL |
| Hydrogen disodium phosphate | 0.7 mg/mL |
| Tween 80 | 0.25 mg/mL |
| Sodium chloride | 7.7 mg/mL |
| Disodium ethylenediamine tetraacetic acid | 0.1 mg/mL |
| Benzalkonium chloride | 0.2 mg/mL |
| Injection water | q.s. 1 ml |

### Example 7:

**Formulation based on rhEPO with low content of sialic acid for intranasal administration, similar to example 2 but with methylparaben and propylparaben as antimicrobial preservative agents.**

### Preparation method:

The preparation of the solution starts from a volume of injection water representing 30% of the total volume of the formulation. This volume is used to dissolve the buffer and the isotonic agent. Apart from this, in a container of appropriate capacity, a volume of injection water equal to 15% of the total volume of the formulation is placed and warmed to a temperature ranging from 90 to 95°C. The parabens are dissolved and the polymer is then incorporated with strong, constant shaking. Once the polymer is moistened, the previously prepared solution is incorporated to the same while shaking vigorously until total homogenization. Shaking is then reduced in order to incorporate the corresponding volume of basic rhEPO, the non-ionic surfactant and the histidine hydrochloride. Finally, the volume is completed with injection water until the 100% of the formulation's final volume. The formulation is subsequently filtered through a membrane of cellulose acetate of 0,2 µm and the pH is tested to verify that it keeps within the established range.

The composition of the finished preparation is as follows:

| **Component** | **Proportion** |
|---|---|
| Basic rhEPO | 0,8 mg/mL |
| Hydroxypropylmethylcellulose | 5,0 mg/mL |
| Histidine hydrochloride | 1,0 mg/mL |
| Dihydrogen sodium phosphate | 2 mg/mL |
| Hydrogen disodium phosphate | 0,7 mg/mL |
| Tween 80 | 0,25 mg/mL |
| Sodium chloride | 7,5 mg/mL |
| Methylparaben | 0,33 mg/mL |
| Propylparaben | 0,17 mg/mL |
| Injection water | q.s. 1 ml |

### Example 8:

**Nasal formulation of rhEPO with low content of sialic acid similar to the former example, but using methylcellulose as bioadhesive polymer.**

### Preparation method: similar to example 6).

The composition of the finished form is as follows:

| **Component** | **Proportion** |
|---|---|
| Basic rhEPO | 0,8 mg/mL |
| Methylcellulose | 3,0 mg/mL |
| Histidine hydrochloride | 1,0 mg/mL |
| Dihydrogen sodium phosphate | 2 mg/mL |
| Hydrogen disodium phosphate | 0,7 mg/mL |
| Tween 80 | 0,25 mg/mL |
| Sodium chloride | 7,5 mg/mL |
| Methylparaben | 0,33 mg/mL |
| Propylparaben | 0,17 mg/mL |
| Injection water | q.s. 1 ml |

### Example 9:

**Nasal formulation of rhEPO with low content of sialic acid similar to example 7, but using Tween 20 as the non-ionic surfactant.**

### Preparation method: similar to example 7.

The composition of the finished preparation is as follows:

| **Component** | **Proportion** |
|---|---|
| Basic rhEPO | 0,8 mg/mL |
| Hydroxypropylmethylcellulose | 5,0 mg/mL |
| Histidine hydrochloride | 1,0 mg/mL |
| Dihydrogen sodium phosphate | 2 mg/mL |
| Hydrogen disodium phosphate | 0,7 mg/mL |
| Tween 20 | 0,20 mg/mL |
| Sodium chloride | 7,5 mg/mL |
| Methylparaben | 0,33 mg/mL |
| Propylparaben | 0,17 mg/mL |
| Injection water | q.s. 1 ml |

### Example 10:

**Formulation based on rhEPO with low content of sialic acid similar to former example, but without histidine hydrochloride as stabilizer and using glucose as isotonic agent.**

### Preparation method: similar to example 7.

The composition of the finished preparation is as follows:

| **Component** | **Proportion** |
|---|---|
| Basic rhEPO | 1,2 mg/mL |
| Hydroxypropylmethylcellulose | 5,0 mg/mL |
| Dihydrogen sodium phosphate | 2 mg/mL |
| Hydrogen disodium phosphate | 0,7 mg/mL |
| Tween 20 | 0,20 mg/mL |
| Glucose | 48,0 mg/mL |
| Methylparaben | 0,33 mg/mL |
| Propylparaben | 0,17 mg/mL |
| Injection water | q.s. 1 ml |

### Example 11:

**Formulation based on rhEPO with low content of sialic acid similar to former example, but with Tween 80 as non-ionic surfactant.**

### Preparation method: similar to example 7.

The composition of the finished preparation is as follows:

| **Component** | **Proportion** |
|---|---|
| Basic rhEPO | 1,2 mg/mL |
| Hydroxypropylmethylcellulose | 5,0 mg/mL |
| Dihydrogen sodium phosphate | 2 mg/mL |
| Hydrogen disodium phosphate | 0,7 mg/mL |
| Tween 80 | 0,25 mg/mL |
| Glucose | 48,0 mg/mL |
| Methylparaben | 0,33 mg/mL |
| Propylparaben | 0,17 mg/mL |
| Injection water | q.s. 1 ml |

### Example 12:

**Nasal formulation of rhEPO with low content of sialic acid similar to example 7, but using chlorobutanol as antimicrobial preservative agent.**

### Preparation method: similar to example 7.

The composition of the finished preparation is as follows:

| **Component** | **Proportion** |
|---|---|
| Basic rhEPO | 0,8 mg/mL |
| Hydroxypropylmethylcellulose | 5,0 mg/mL |
| Histidine hydrochloride | 1,0 mg/mL |
| Dihydrogen sodium phosphate | 2 mg/mL |
| Hydrogen disodium phosphate | 0,7 mg/mL |
| Tween 80 | 0,25 mg/mL |
| Sodium chloride | 6,2 mg/mL |
| Chlorobutanol | 5,0 mg/mL |
| Injection water | q.s. 1 ml |

### Example 13:

**Formulation based on rhEPO with low content of sialic acid similar to former example, but with methylcellulose as bioadhesive polymer.**

### Preparation method: similar to example 2.

The composition of the finished preparation is as follows:

| **Component** | **Proportion** |
|---|---|
| Basic rhEPO | 0,8 mg/mL |
| Methylcellulose | 3,0 mg/mL |
| Histidine hydrochloride | 1,0 mg/mL |
| Dihydrogen sodium phosphate | 2 mg/mL |
| Hydrogen disodium phosphate | 0,7 mg/mL |
| Tween 80 | 0,25 mg/mL |
| Sodium chloride | 6,2 mg/mL |
| Chlorobutanol | 5,0 mg/mL |
| Injection water | q.s. 1 ml |

### Example 14:

**Nasal formulation of rhEPO with low content of sialic acid similar to example 12, but using Tween 20 as the non-ionic surfactant.**

### Preparation method: similar to example 2.

The composition of the finished preparation is as follows:

| **Component** | **Proportion** |
|---|---|
| Basic rhEPO | 0,8 mg/mL |
| Hydroxypropylmethylcellulose | 5,0 mg/mL |
| Histidine hydrochloride | 1,0 mg/mL |
| Dihydrogen sodium phosphate | 2 mg/mL |
| Hydrogen disodium phosphate | 0,7 mg/mL |
| Tween 20 | 0,20 mg/mL |
| Sodium chloride | 6,2 mg/mL |
| Chlorobutanol | 5,0 mg/mL |
| Injection water | q.s. 1 ml |

### Example 15:

**Nasal formulation of rhEPO with low content of sialic acid similar to example 5, but with Chlorobutanol as antimicrobial preservative.**

### Preparation method: similar to example 2.

The composition of the finished preparation is as follows:

| **Component** | **Proportion** |
|---|---|
| Basic rhEPO | 1,2 mg/mL |
| Hydroxypropylmethylcellulose | 5 mg/mL |
| Dihydrogen sodium phosphate | 2 mg/mL |
| Hydrogen disodium phosphate | 0,7 mg/mL |
| Tween 20 | 0,20 mg/mL |
| Sodium chloride | 6,5 mg/mL |
| Chlorobutanol | 5,0 mg/mL |
| Injection water | q.s. 1 ml |

### Example 16:

**Evaluation *"in vitro"* of the stimulating action on cells of nervous origin of the nasal formulation of recombinant human erythropoietin with a low content of sialic acid.**

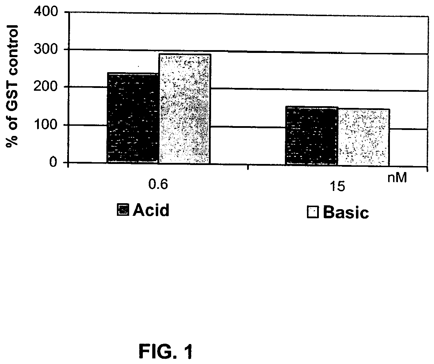

In order to demonstrate the inductive effect on the specific activity of the enzyme Glutation-S-Transferase (GST) of rhEPO on cells of nervous origin, as well as to demonstrate that acid and basic recombinant human erythropoietin have similar effects on the cells of nervous origin, a study in vitro was conducted on PC12 cells. The cells were kept undifferentiated in DMEM medium supplemented with 2 mM of L-Glutamine, 100 Units/ml penicillin G, 100 micrograms/ml streptomycin (GIBCO), 10 % (v/v) containing 10 mM of Hepes buffer. The culture medium was prepared with whole horse serum at 10 %. The cells were added 0,6 and 15 nanomolar recombinant human erythropoietin, either with low content of sialic acid (examples 5 and 6) or acid (fully glycosilated) erythropoietin. As controls, cells were added to similar quantities of rhEPO, previously inactivated by heat. The values of specific activity of the GST obtained in these cultures were considered as 100 %. For the determination of the specific activity of the GST, the homogenate of the cells cultivated with acid or basic rhEPO was used; this was carried out in a homogenization system entirely made with glass at 4 °C, with 0,1 M phosphate buffer, pH = 6,95. The homogenate free from cells was obtained by centrifugation at 16 000 X g, at 4 degrees Celsius, in a refrigerated high-speed centrifuge. The homogenate thus obtained was kept in an iced-water bath and was used for the determination of the specific activity of the GST according to the method of Habig. (Habig, W.H., Pabst, J.J., and Jakoby, W.B. 1974; Biol. Chem. 249 (22): 7130-7139). The determination of protein was carried out by the method of Bradford (Bradford Anal MM. Biochem, 72, (176) 158). The main result obtained was that the cells of nervous origin responded to the presence of rhEPO by modifying the activity of the enzyme GST. The values in the graph (Fig. 1) represent the mean value of three independent cultures. The response to recombinant human erythropoietin with low content of sialic acid was similar to the response to acid rhEPO.

### Example 17:

**Therapeutic effectiveness of intranasal administration of rhEPO with low content of sialic acid.**

Mongolia gerbils of both sexes with body weight between 60 and 70 grams were used. They were taking water and feed *ad libitum* and were maintained on cycles of 12 light hours alternating with 12 darkness hours during the whole experimental period.

The animals underwent permanent unilateral occlusion of the right carotid under deep anesthesia by the intraperitoneal way, with diazepam (5,0 mg/kg) as pre-anesthetic. Ketamine (47 mg/kg) and atropine (0,02 mg/kg) were used as the anesthetic agent. The right carotid was exposed under stereoscope, doubly ligated and cut. The sham animals were prepared with the same manipulations but without ligating or cutting of the carotid.

For the clinical and histopathological evaluation, the experimental groups were: Control group: carotid isolated, without any other procedure (sham) (n = 10). Group of injured animals without treatment: Animals injured, without having undergone any other procedure (n = 20).
Group of injured animals with treatment: carotid isolated, doubly ligated and cut, with intranasal administration of rhEPO with low content of sialic acid (example 10) (n = 20).

The treatment consisted in the application into the nasal cavity of 10 µl of rhEPO or its vehicle every 8 hours daily, from an hour after the operation until the 4th day subsequent to the operation.

Each animal was examined to determine its neurological status according to a scale from 2 to 5 that included corporal tone, prehensile force and alterations in posture and walking. A surviving animal without pathological signs has a value of 5 in this scale.

The functional evaluation was performed by observing the spontaneous exploratory activity of the animals, that is, by counting the number of body rearing actions (i.e., the instances of standing on the tiptoes of the rear paws) made by the rodents when exploring a new recipient. The recipient used was a vertical cylindrical box 30 cm in diameter and 25 cm high. Each gerbil was placed at the center of this box and the rearing actions performed during an interval of 3 minutes were counted.

Seven days after the operation, the animals were perfused through the left ventricle with 4 % formaldehyde solution in phosphate buffer solution (PBS) at pH = 7,0. The brains were extracted and kept in this solution for some days. Later on the brains were included in paraffin, cut into slices 4 µm thick and colored with hematoxylin and eosin. The sections were evaluated at 10 x and 40 x without previous knowledge of their identity.

For the evaluation of cerebral edema, 90 females between 60 and 70 g body weight were used, comprised in the 3 experimental groups:
Sham animals (n = 20).
Injured animals without treatment (n = 35).
Injured animals with treatment (n = 35)

Three, 12 or 24 hours after the injury the animals were anesthetized and perfused with saline solution. The brain was extracted from the cranial cavity and the hemispheres were separated from each other. The determination of water content was carried out according to the gravimetric method described by Calapai and cols. 2000 according to the equation: Water % = [(Humid Weight of the Hemisphere - Dry Weight of the Hemisphere) X 100 X Humid Weight ⁻¹]. For the analysis of the data, differences were established between groups and samplings for each variable by means of the Student's t test, the ANOVA (one way test) and the Dunnett's test for Multiple Comparison.

For the evaluation of the body weight of the animals, males were randomly assorted into three experimental groups (10 animals each group) by following the same treatment outline used in the previous experiments. The animals were weighed by using a Sartorius scale on the day of the operation and later on for 5 serial weeks (1 time per week).

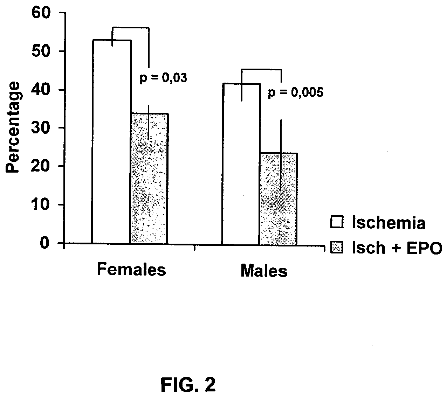

During the experiments described with nasal administration of rhEPO, a lower mortality rate was observed among the treated animals during the days subsequent to the surgery in both sexes, as evidenced by the analysis of the proportions (Fig. 2).

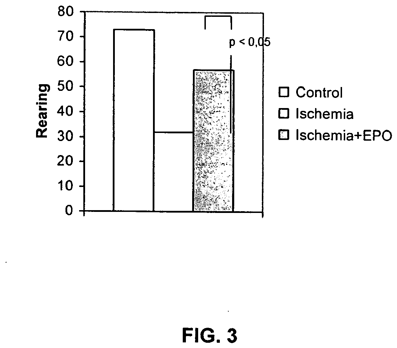

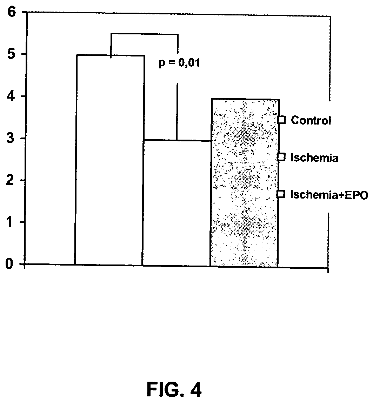

Twenty-four hours after the unilateral ligature, a part of the animals showed affectations in the neurological status, revealed by means of the exam described and expressed by the score (Fig. 3). As it can be observed, in the animals injured without treatment a significant functional injury appeared. The motor-functional exploratory activity appeared depressed in non-treated ischemic animals (Fig. 4), while this activity in the animals treated with rhEPO remained similar to that of the controls.

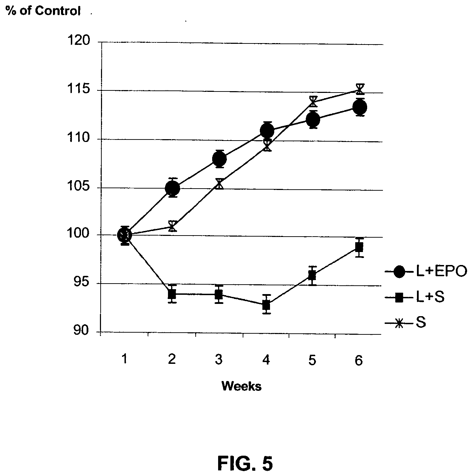

The effects of injury and intranasal administration of rhEPO on the body weight of the animals in the model of permanent unilateral ischemia are shown in Fig. 5. A comparison between the group of injured animals with the injured and treated group and with the sham group showed different behaviors of the weight curves. The control group and the injured treated group had similar curves, contrasting with the loss of weight in the injured, non-treated animals. In the course of several weeks, this group could not surpass the average weight with which the experiment began.

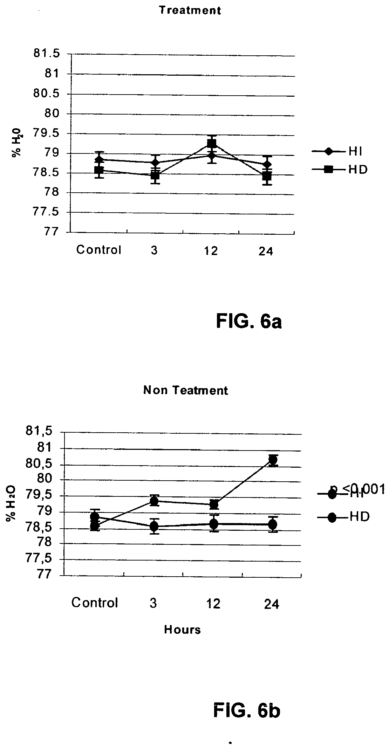

The results consisting in a decrease of brain edema were due to the fact that intranasal administration of rhEPO provided significant protection from the formation of cerebral edema in all the animals treated (Fig. 6, a and b). In fact, no differences in water content were found between the animals treated with rhEPO and the controls. A diametrically opposed situation was found in the group of injured, non-treated animals, in which the water content in the injured (right) hemisphere had increased for all the times studied (3, 12 and 24 hours), and a high significance for 24 hours after the injury was found (p < 0,001).

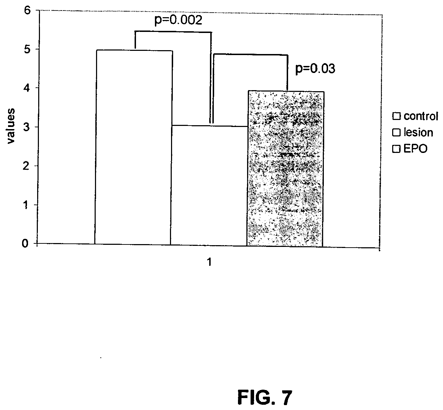

As regards the histopathological findings, the frequency of animals with the brain intact was greater among the control animals than among the non-treated animals (p = 0,002), and it was also greater among the animals treated with rhEPO than among the non-treated animals (p = 0,03), (Fig. 7).

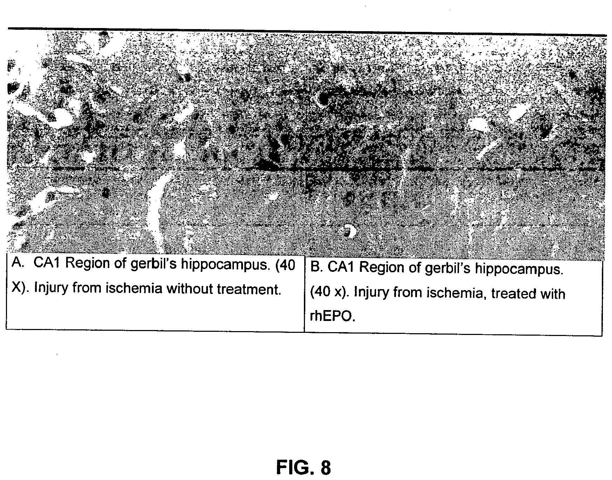

The non-treated animals were affected by generalized edema and pyknosis of the whole right hemisphere, as well as by hemorrhages on the fimbria of the right hippocampus and on the parietal cortex of the right hemisphere, edematous areas and dense, diffuse chromatin in the right hemisphere, pyknotic neurons in the hippocampus (Fig. 8).

### Example 18:

**Evaluation *in vitro* of the antioxidant capacity of basic recombinant human erythropoietin with low content of sialic acid (example 10) in homogenate of anatomical regions of hippocampus, cerebral cortex and cerebellum of the rat.**

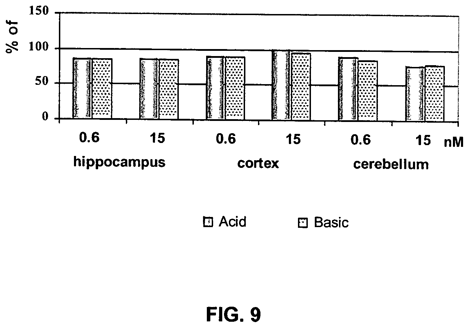

In order to demonstrate that both acid and basic rhEPO are able to counteract the production of malondialdehyde (MDA) and 4-hydroxyalkenals (4HDA) in homogenate of rat brain, an experiment *in vivo* was conducted. For this purpose, male Wistar rats (n = 15) with body weight between 180 and 200 grams were used. The animals were taking water and feed *ad libitum* and were under a regime of 12 light hours alternating with 12 darkness hours during the whole experimental period. The methodology applied to obtain the anatomical regions was as follows:
The animals were sacrificed by cervical dislocation and decapitation, the brain was quickly removed from the cranial cavity and the tissue was immediately frozen to - 70 degrees Celsius. The hippocampus, the cerebral cortex and the cerebellum of each animal were dissected with help of a stereotaxic atlas (Paxinos, G., and Watson, C., 1986. Academic Press, New York. pp 230-259). A pool of each one of these regions was formed and a homogenate of each region was prepared, which contained 1:8 parts of tissue per part of phosphate buffer 0,1 M at pH = 7,0. The liquid free from cells was obtained by centrifugation at 4 degrees Celsius at 10 000 g for 30 minutes. The homogenates were kept in a bath of iced water until the determination of the enzymatic activity, for which a Spectro UV-VIS RS digital spectrophotometer from Labo Med, Inc. was used. The main result obtained was that recombinant human erythropoietin with low content of sialic acid reduced by 13 % (0 - 23 %) the production of MDA + 4 HDA in the homogenates of the three regions studied of the brain of the rat. The decrease was dependent from the dose used. The highest levels in protection were observed in the hippocampus and the cerebellum, where considerable levels of rhEPO receivers have been detected. The responses of acid and basic rhEPO were similar (Fig.9).

### Example 19:

**The application of 90 000 IU of recombinant human erythropoietin with low content of sialic acid (according to formulation in example 6) does not induce a significant erythropoietic response.**

In order to demonstrate that the basic recombinant human erythropoietin with low content of sialic acid used in the trials does not have any significant erythropoietic activity *in vivo*, its effects on the erythropoiesis of the Mongolia gerbils were assessed using a dose of 90 000 IU in a comparative study with 10 animals (5 with administration of rhEPO and 5 controls). All the animals were monitored (before the administration, 7 days post administration and 14 days post administration) to carry out a complete hemogram in total blood (10 µL EDTA at 10 %/mL of blood) using a *Micros ABX* automatic counter for processing the samples. The statistical package SPSS based on Windows, taking p = 0,05 as confidence level, was used for processing the variables. The following results were obtained (Table 1).

**Table 1. Hematological parameters obtained for the different groups.**

| | | ***SAMPLINGS*** | | | | | |
|---|---|---|---|---|---|---|---|
| | | **1** | | **2** | | **3** | |
| | | **X** | **SD** | **X** | **SD** | X | **SD** |
| ***GROUP 1*** | HB (g/dL) | 13,3 | 1,1 | 13,2 | 0,7 | 12,8 | 0,9 |
| | ETO (10⁶/µL) | 7,82 | 0,95 | 8,04 | 0,54 | 7,63 | 0,59 |
| | HTC (%) | 41,9 | 4,5 | 43,2 | 2,8 | 41,4 | 3,6 |
| | PLT (10³/µL) | 612 | 214 | 670 | 101 | 761 | 75 |
| | VCM (fL) | 53 | 1 | 54 | 1 | 54 | 1 |
| | HCM (pg) | 17,0 | 0,7 | 16,4 | 0,3 | 16,8 | 0,2 |
| | CHCM (g/dL) | 31,7 | 0,9 | 30,5 | 0,5 | 31,0 | 0,6 |
| | LEUC (10³/µL) | 9,7 | 4,9 | 8,8 | 3,6 | 6,0 | 2,8 |
| | RETIC (%) | 1,6 | 0,7 | 1,9 | 0,8 | 3,0 | 1,3 |
| | RETIC (10⁶/µL) | 0,12 | 0,05 | 0,16 | 0,07 | 0,22 | 0,09 |
| ***GROUP 2*** | HB (g/dL) | 13,4 | 0,5 | 14,3 | 0,3 | 13,3 | 0,3 |
| | ETO (10⁶/µL) | 8,21 | 0,55 | 8,82 | 0,41 | 8,13 | 0,45 |
| | HTC (%) | 43,4 | 2,4 | 48,1 | 1,1 | 43,9 | 1,7 |
| | PLT (10³/µL) | 503 | 213 | 673 | 197 | 714 | 134 |
| | VCM (fL) | 53 | 2 | 55 | 2 | 54 | 2 |
| | HCM (pg) | 16,4 | 0,9 | 16,2 | 0,6 | 16,4 | 0,8 |
| | CHCM (g/dL) | 31,0 | 0,9 | 29,7 | 0,5 | 30,4 | 0,7 |
| | LEUC (10³/µL) | 5,4 | 1,2 | 4,9 | 0,9 | 5,1 | 0,9 |
| | RETIC (%) | 1,3 | 0,6 | 1,6 | 0,5 | 2,1 | 1,3 |
| | RETIC (10⁶/µL) | 0,11 | 0,05 | 0,14 | 0,04 | 0,17 | 0,10 |

The distribution of the populations for each hematological parameter by sampling with p = 0,05 and n = 10 showed a normal distribution. In the analysis of variance homogeneity it was observed that, in a general way, the variances had a homogeneous behavior, except HB and HTC in sampling I, that is, LEUC.

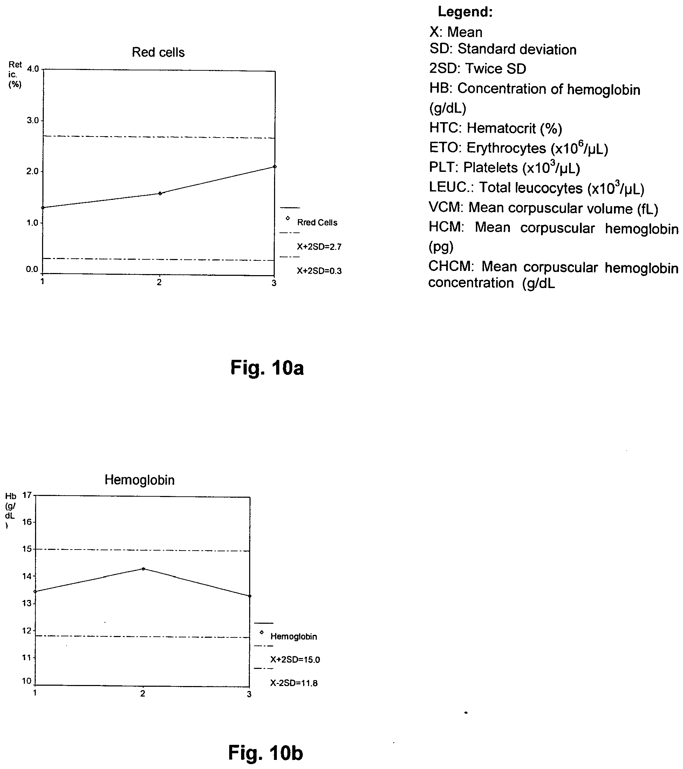

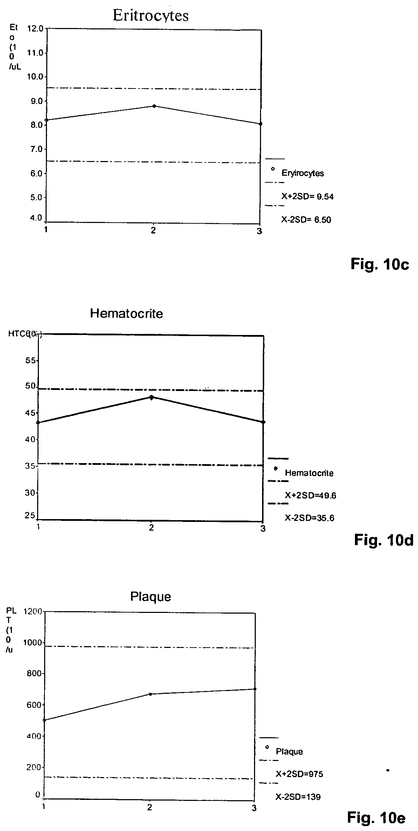

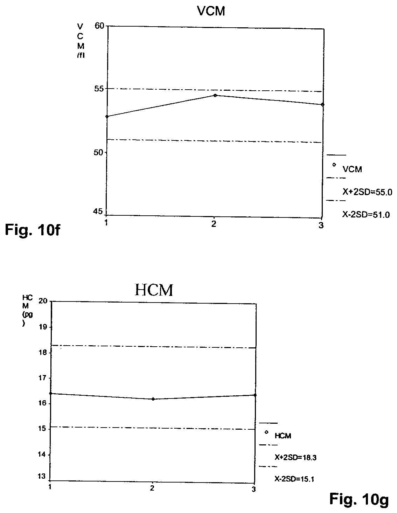

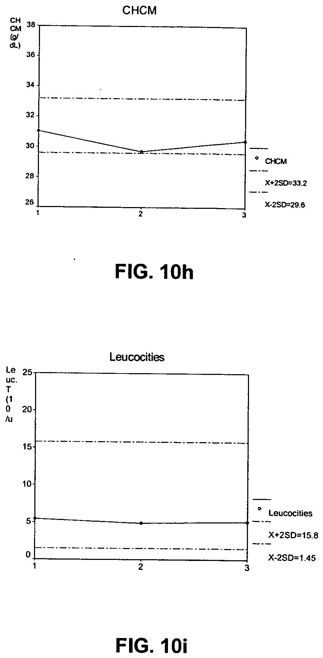

On comparing the variances of the hematological variable of the groups in the different samplings, differences were observed in HB, ETO, HTC and CHCM of sampling II, where differences are evidenced between the control group and the treated group. On comparing the variances between the samplings for each group, differences were observed in CHCM of the control group (sampling I vs. sampling II), whereas the treated group showed differences in HB and HTC (sampling II vs. samplings I and III) and the CHCM (sampling II vs. sampling I). For the evaluation of the differences of the aforementioned variables between the groups by samplings, sampling I was taken as reference sampling for the comparisons. Keeping in mind that some statistical differences p = 0,05 are shown between the groups and between the samplings, it was decided to carry out a more integral evaluation of the data obtained; for this purpose, the values of sampling I were grouped, a moment in which the animals had not yet been treated n = 10, and the range of this population X ± 2 SD was calculated, then the behavior of the mean values for each variable was graphed (Fig. 10). The mean values of the variables were also compared with those reported by other authors for this species, and then it was ascertained that, in all the groups and samplings, the values obtained were similar to those reported in the literature (Table 2).

Even though the statistical comparisons show that the variables HB, ETO and HTC increased significantly in sampling II, on analyzing this behavior according to the normal range X ± 2 SD and the mean values reported in the literature (Handbook of Care and Use of Experimental Animals, Canadian Council for Animal Protection. Volume 1, 2nd edition. 1998), one can notice that this behavior lacks biological significance, so that one can conclude that recombinant human erythropoietin with low content of sialic acid does not induce any significant erythropoietic response in the species studied.

**Table 2. Normal hematological values for gerbils.**

| Parameter | Normal hematological values for gerbils |
|---|---|
| ERIT (10⁶/µL) | 8,5 (7,0-10,0) |
| Hb (g/dL) | 15,0 (12,1-16,9) |
| PCV (L/L) | 0,48 (0,41-0,52) |
| PLT (10³/µL) | 638 |
| LEUC. T (10³/µL) | 4,3-21,6 |
| Neutrophiles (10³/µL) | 0,3-4,1 |
| Lymphocytes (10³/µL) | 3,2-9,7 |
| Blood V. (mg/kg) | 60-85 |

### Example 20:

**Labeling with I¹²⁵ of recombinant human erythropoietin with low content of sialic acid.**

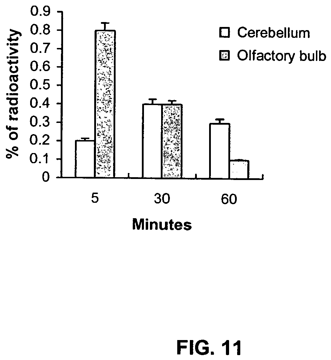

In order to determine the passage of recombinant human erythropoietin with low content of sialic acid (examples 3 and 5) to regions of the central nervous system by intranasal administration, rhEPO was marked with iodine 125 according to the commercial method (YODOGEN) from Pierce, Rockford, Illinois 61105, USA. Starting from this radioactive marking, intranasal formulations were prepared. A total of 18 male Wistar rats of body weight between 180 and 200 g were used. The animals were treated by intranasal administration of this formulation of recombinant human erythropoietin with low content of sialic acid labeled with I¹²⁵. Each animal received intranasal administration of 5 to 100 microliters of the formulation. At intervals of 5, 30 or 60 minutes the animals were sacrificed by decapitation under anesthesia. The brain was removed quickly (in less than 70 seconds). The areas of the olfactory bulb and the cerebellum were extracted. The radioactive count was carried out with a gamma counter. The following results were obtained: An application of recombinant human erythropoietin with low content of sialic acid reaches regions of the brain in at least 5 minutes and it diminishes with time in a gradual way from the frontal to the caudal region of the brain. Upon 60 minutes from the application, there is still some rhEPO in the regions of the olfactory bulbs and the cerebellum. These results indicate that approximately between 1 and 5 % of nasally applied rhEPO reaches the brain. Between 80 and 85 % of the rhEPO reaching the brain was detected in the olfactory bulbs, while nearly 20 % was detected in the cerebellum upon 5 minutes from the application. After some 30 minutes these concentrations were almost equal, and after 60 minutes the cerebellum had 30 % and only 10 % of the radioactivity of the olfactory bulbs. The presence of the marked molecule in regions of the cerebellum indicates the passage of BBB; on the other hand, the detection of the molecule at the level of regions not related with the olfactory way (cerebellum) indicates that not only does the olfactory way take part in the passage of molecules to the brain, but also does a more general, faster mechanism, perhaps the diffusion through the mucus and subsequent permeation through discontinuities existing particularly in regions of the olfactory epithelium in the area of the *macula cribosa*, which allows the arrival of recombinant human erythropoietin with low content of sialic acid to regions far from the olfactory way. Fig. 11 represents the passage of recombinant human erythropoietin with low content of sialic acid from the nasal cavity to the olfactory bulb and cerebellum. Each bar in the figure represents the average value of six animals. The figure shows how the recombinant human erythropoietin with low content of sialic acid reaches regions of the brain in at least 5 minutes and diminishes with time in a gradual way from the frontal to the caudal region of the brain. Upon 60 minutes from the application, there is still some rhEPO in the regions of the olfactory bulb and the cerebellum. Between 80 and 85 % of the rhEPO reaching the brain was detected in the olfactory bulbs, while nearly 20 % was detected in the cerebellum upon 5 minutes from the application. After some 30 minutes these concentrations were almost equal, and after 60 minutes the cerebellum had 30 %, and only 10 % of the radioactivity of the olfactory bulbs.

### Example 21:

**Effect of basic rhEPO on subarachnoid hemorrhage in rabbits.**

The trial was carried out on male New Zealand rabbits weighing between 3 and 4 kg, which were anesthetized with intramuscular injection of ketamine 40 mg/kg. Later on, 5 ml of blood were extracted from the central artery of the ear and were injected by the percutaneous way into the great cistern. The animals were monitored for 72 hours.

The rabbits were divided into 3 experimental groups with 8 animals each:
Group I: Control Animals.
Group II: Animals injured.
Group III: Animals injured + basic rhEPO 20 µg (example 5).

The administration of basic rhEPO was carried out 5 minutes after inducing the subarachnoid hemorrhage and was repeated every 8 hours during 72 hours.

For the neurological evaluation, the motor-functional behavior of the animals was followed according to Grasso G, 2002 during the period of trial.

The results are shown in Table I. A better motor-functional behavior was observed among the control animals (without injury) and the animals treated with basic rhEPO than among the injured, non-treated animals.

| Neurological evaluation observed 72 hours after the induced hemorrhage | | | | |
|---|---|---|---|---|
| Groups | Scale of neurological evaluation | | | |
| | **1** | **2** | **3** | **4** |
| Control Animals | 8 | 0 | 0 | 0 |
| Animals injured * | 0 | 0 | 2 | 6 |
| Animals injured + basic rhEPO | 6 | 2 | 0 | 0 |

| | | | | |
|---|---|---|---|---|
| The degree of neurological injury was classified as: 1: No indications of injury. 2: Minimum injury. 3: Moderate injury without abnormal movements. 4: Severe injury with presence of abnormal movements. *: There was significant difference between the animals injured and treated with basic rhEPO and those injured for p < 0,05. | | | | |

### Example 22:

### Effect of basic rhEPO for the treatment of dementia

In 36 Mongolian gerbils the right carotid was tied under anesthesia. The animals received intranasal administration of 40 IU of rhEPO (examples 10 and 11) daily during 7 days, beginning within the time space of 1 hour subsequent to surgery. Other 24 animals undergoing a similar processing received an equivalent quantity of vehicle. Nine gerbils undergoing isolation of the carotid without ligature constituted the control group.

Eight days after the operation, 27 and 13 animals had survived among the groups subjected to unilateral ischemia and treated with rhEPO and those treated with vehicle only, respectively. There was not mortality for the control group.

The animals were subjected to a habituation test one day before and one week after the unilateral ligature of the carotid. This test is based on the count of rearing actions in a cylindrical box during an interval of 9 minutes divided into three thirds. The proportions of the rearing actions in each third determine a straight line of best fit between the three points. The slope was used to establish comparisons between groups by means of the Mann and Whitney's U test. The comparisons before-after were carried out by means of Wilcoxon's range comparison test of the surviving animals. The results were as follows:

| Group | Before | After | Value of p |
|---|---|---|---|
| Controls | -14,2 | -12,3 | 0,2 |
| Vehicle | -16,6 | -7,9 ^{a, b} | 0,01 |
| rhEPO | -15,3 | -14,0 | 0,1 |

| | | | |
|---|---|---|---|
| a - Different from control; b - Different from rhEPO; p < 0,05. | | | |

The results show a smaller slope (closer to 0) for the animals subjected to ischemia, one week after the ischemia and in comparison with those treated with rhEPO and the controls. A smaller slope demonstrates a persistence of the animal in exploring its surroundings in the latter two thirds of the 9-minute interval, showing a loss of the habituation that can be characterized as a cognitive dysfunction induced by the ischemia. The treatment with basic rhEPO prevents the appearance of this dysfunction.

The behavior of the animals revealed a cerebral deterioration, induced by the ischemia, which could be expressed as a loss of the capacity for nesting, which is typical in the males of this species; also as an increment of aggressiveness and loss of the self-grooming behavior during the time they were kept in the circular cylinder. The results were compared by means of the chi-squared independence test and are expressed in the table by percentage:

| Group | Nesting | Aggressiveness | Self-grooming |
|---|---|---|---|
| Control | 88 | 11 | 88 |
| Vehicle | 23 ^{a, b} | 92 ^{a, b} | 30 ^{a} |
| rhEPO | 77 | 44 ^{a} | 66 |

| | | | |
|---|---|---|---|
| a - Different from control; b - Different from rhEPO; p < 0,05 | | | |

The animals were sacrificed by perfusion under anesthesia with neuter formalin buffered at 10 %. The brains were extracted and processed for obtaining coronal sections 8 µm thick colored with hematoxylin and eosin. The sections were examined at a clear-field microscope for histopathological signs. The comparisons were carried out by means of the chi-squared independence test. The results in the table are expressed in percentage:

| Group | Pyknosis | Gliosis | Edema | Neuron loss |
|---|---|---|---|---|
| Control | 22 | 0 | 0 | 0 |
| Vehicle | 100 ^{a} | 92 ^{a, b} | 23 ^{a, b} | 69 ^{a, b} |
| Basic rhEPO | 55 | 44 ^{a} | 0 | 33 ^{a} |

| | | | | |
|---|---|---|---|---|
| a - Different from control; b - Different from rhEPO; p < 0,05. | | | | |

It is remarkable that the treatment prevents the edema totally and reduces the incidence of pyknosis, gliosis and neuronal loss by one half.

The behavioral deterioration can be qualified as a reflection of the neuronal loss and of other histopathological findings observed in the non-treated group. The treatment with rhEPO prevented the behavioral deterioration induced by the decrease of the cerebral blood flow in the Mongolia gerbil, at least partially. The results are excellent in the sense of suggesting a neuron-protecting action of rhEPO that is able to counteract the functional deterioration consisting in dementia of vascular origin in humans.

## Claims

1. Nasal formulation comprising recombinant human erythropoietin, wherein less than 40% of the erythropoietin molecule is protected with sialic acid and the concentration of the active protein is in the range of about 0.5 and 2.0 mg/ml.

2. Nasal formulation according to claim 1 **characterized by** the fact that the finished form is nose drops.

3. Nasal formulations according to claim 1 **characterized by** the fact that the finished form is a nasal spray.

4. Nasal formulation according to claim 1, 2 or 3 **characterized by** the use of bioadhesive polymers such as hydroxypropylmethylcellulose (HPMC), hydroxypropylcellulose (HPC) and methylcellulose (MC).

5. Nasal formulation according to claim 1, 2 or 3 **characterized by** the use of antimicrobial preservatives such as benzalkonium chloride, chlorobutanol, methylparaben and propylparaben.

6. Nasal formulation according to claim 1, 2 or 3 **characterized by** the use of protein stabilizers such as L- tryptophane, L- leucine, L-arginine hydrochloride and/or L-histidine hydrochloride and/or their salts.

7. Nasal formulation according to any one of claims 1-6 for use in medicine.

8. Nasal formulation for use according to claim 7, wherein the formulation is for use in the treatment or prevention of central nervous system disorders such as cerebrovascular or neurodegenerative disorders, hypoxia of the newborn, hypoxia from a cardiovascular cause, dementia, loss of memory, decrease of mental capacity and mental deterioration.

9. Recombinant human erythropoietin, wherein less than 40% of the erythropoietin molecule is protected with sialic acid, for use in the treatment of central nervous system disorders such as cerebrovascular and neurodegenerative disorders; wherein said treatment involves intranasal administration of said erythropoietin.

## Patentansprüche

1. Nasale Formulierung, umfassend rekombinantes menschliches Erythropoietin, wobei weniger als 40 % des Erythropoietinmoleküls durch Sialinsäure geschützt ist und die Konzentration des aktiven Proteins im Bereich von ungefähr 0,5 und 2,0 mg/ml ist.

2. Nasale Formulierung nach Anspruch 1, **gekennzeichnet durch** die Tatsache, dass die fertige Form Nasentropfen ist.

3. Nasale Formulierung nach Anspruch 1, **gekennzeichnet durch** die Tatsache, dass die fertige Form ein Nasenspray ist.

4. Nasale Formulierung nach Anspruch 1, 2 oder 3, **gekennzeichnet durch** die Verwendung von bioadhäsiven Polymeren wie etwa Hydroxypropylmethylzellulose (HPMC), Hydroxypropylzellulose (HPC) und Methylzellulose (MC).

5. Nasale Formulierung nach Anspruch 1, 2 oder 3, **gekennzeichnet durch** die Verwendung antimikrobieller Präservative wie etwa Benzalkoniumchlorid, Chlorobutanol, Methylparaben und Propylparaben.

6. Nasale Formulierung nach Anspruch 1, 2 oder 3, **gekennzeichnet durch** die Verwendung von Proteinstabilisatoren wie etwa L-Tryptophan, L-Leucin, L-Arginnhydrochlorid und/oder L-Histidinhydrochlorid und/oder deren Salzen.

7. Nasale Formulierung nach einem der Ansprüche 1-6 zur Verwendung in der Medizin.

8. Nasale Formulierung zur Verwendung nach Anspruch 7, wobei die Formulierung zur Verwendung in der Behandlung oder Verhinderung von Erkrankungen des zentralen Nervensystems ist, wie etwa zerebrovaskulären oder neurodegenerativen Erkrankungen, Hypoxie bei Neugeborenen, Hypoxie durch kardiovaskuläre Ursachen, Demenz, Gedächtnisverlust, Nachlassen der geistigen Leistungsfähigkeit und geistigen Verfalls.

9. Rekombinantes menschliches Erythropoietin, wobei weniger als 40 % des Erythropoietinmoleküls durch Sialinsäure geschützt ist, zur Verwendung in der Behandlung von Erkrankungen des zentralen Nervensystems wie etwa zerebrovaskuläre und neurodegenerative Erkrankungen; wobei die Behandlung die intranasale Verabreichung des Erythropoietins einschließt.

## Revendications

1. Formulation à usage nasal comprenant de l'érythropoïétine humaine recombinée, dans laquelle moins de 40 % de la molécule d'érythropoïétine sont protégés avec de l'acide sialique et la concentration de la protéine active est située dans la plage allant d'environ 0,5 à 2,0 mg/ml.

2. Formulation à usage nasal selon la revendication 1, **caractérisée en ce que** la forme finale est des gouttes nasales.

3. Formulation à usage nasal selon la revendication 1, **caractérisée en ce que** la forme finale est un spray à usage nasal.

4. Formulation à usage nasal selon la revendication 1, 2 ou 3, **caractérisée par** l'utilisation de polymères bioadhésifs tels que l'hydroxypropylméthylcellulose (HPMC), l'hydroxypropylcellulose (HPC) et la méthylcellulose (MC).

5. Formulation à usage nasal selon la revendication 1, 2 ou 3, **caractérisée par** l'utilisation de conservateurs antimicrobiens tels que le chlorure de benzalkonium, le chlorobutanol, le méthylparaben et le propylparaben.

6. Formulation à usage nasal selon la revendication 1, 2 ou 3, **caractérisée par** l'utilisation de stabilisants de protéines tels que le L-tryptophane, la L-leucine, le chlorhydrate de L-arginine et/ou le chlorhydrate de L-histidine et/ou leurs sels.

7. Formulation à usage nasal selon l'une quelconque des revendications 1 à 6, pour une utilisation en médecine.

8. Formulation à usage nasal pour une utilisation selon la revendication 7, laquelle formulation est destinée à être utilisée dans le traitement ou la prévention de troubles du système nerveux central tels que des troubles cérébrovasculaires ou neurodégénératifs, l'hypoxie du nouveau-né, une hypoxie ayant une cause cardiovasculaire, une démence, une perte de mémoire, une diminution de la capacité mentale, et une détérioration mentale.

9. Erythropoïétine humaine recombinée, dans laquelle moins de 40 % de la molécule d'érythropoïétine sont protégés avec de l'acide sialique, pour une utilisation dans le traitement de troubles du système nerveux central tels que des troubles cérébrovasculaires ou neurodégénératifs ; dans laquelle ledit traitement met en jeu une administration par voie intranasale de ladite érythropoïétine.
